# EUROPEAN PATENT APPLICATION

(11) **EP 4 647 062 A1**
(43) Date of publication of application: **12.11.2025**
(21) Application number: 24175274.0
(22) Date of filing: 10.05.2024
(51) Int. Cl.: A61K 8/31, A61K 8/37, A61K 8/39, A61K 8/92, A61Q 1/14, A61Q 19/10

(54) **SPONTANEOUSLY EMULSIFYING COMPOSITIONS**

(71) Applicant: Gobiotics BV, 4631 SL Hoogerheide (NL)
(72) Inventor: Gonry, Patrick, 2910 Essen (BE); Piotrowska, Anna, 2170 Merksem (BE)
(74) Representative: De Clercq & Partners

(57) **Abstract**

The present application relates to an emulsifying composition comprising polyglyceryl-3 dioleate, polyglyceryl-3 polyricoleate and polyglyceryl-2 laurate, and to oil-based composition comprising said emulsifying composition. Oil-based cosmetic products comprising an oil and the specific combination of polyglycerol esters form a clear homogeneous blend and spontaneously emulsify upon contact with water. They exhibit no greasy effect after rinsing of the oil-based cosmetic product.

## Description

### Field

The present application generally relates to compositions with emulsifying properties for use in oil-based cosmetic products, such as shower oils, and to spontaneously emulsifying oil-based cosmetic products comprising said compositions with emulsifying properties.

### Background

Oil-based cosmetic products, particularly oil-based skincare products, such as shower oils, are currently very popular. They generally offer a gentle cleansing by maintaining the skin's natural oils, making them particularly suitable for people with sensitive or dry skin. They are usually also formulated to hydrate and nourish the skin, thus combining cleansing and moisturizing properties. It is further desired that such oil-based cosmetic products emulsify into a milky texture when applied to wet skin upon contact with water, an effect referred to as "blooming", and that no greasy residue is left behind.

Often, these oil-based cosmetic products contain ethoxylated emulsifiers to obtain these effects. However, some of these compounds can cause skin irritation and their production and use can have environmental implications as well.

Accordingly, despite the popularity of oil-base cosmetic products, there remains a need to make the formula for such products natural and free of ethoxylated emulsifiers and microplastics, that is applicable to a wide variety of oils or oil mixtures, while retaining the blooming effect..

### Summary

The inventors have developed compositions with emulsifying properties for oil-based cosmetic products, comprising a specific combination of polyglycerol esters, which address one or more of the above needs. Oil-based products comprising the compositions with emulsifying properties according to the present application form a clear homogeneous blend and spontaneously emulsify upon contact with water. There is no greasy effect after rinsing of the oil-based product. The emulsifying composition according to the present application is a perfect replacement for ethoxylated emulsifiers and is applicable to a wide variety of oils.

A first aspect of the present invention provides a composition, comprising a first, a second and a third polyglycerol ester, wherein the first polyglycerol ester is polyglyceryl-3 dioleate, wherein the second polyglycerol ester is polyglyceryl-3 polyricoleate and wherein the third polyglycerol ester is polyglyceryl-2 laurate, and wherein the sum of the first, second and third polyglycerol ester is at least 95 % (w/w) based on the total weight of polyglycerol esters in the composition. In certain embodiments, the composition further comprises an oil, wherein the sum of the oil and the first, second and third polyglycerol ester is at least 90 % (w/w), or at least 95 % (w/w), based on the total weight of the composition.

In particular embodiments, the composition comprises between 25.0 % (w/w) and 90.0 % (w/w) of an oil; between 2.0 % (w/w) and 30.0 % (w/w) of the first polyglycerol ester; between 1.0 % (w/w) and 25.0 % (w/w) of the second polyglycerol ester; and between 4.0 % (w/w) and 45.0 % (w/w) of the third polyglycerol ester.

In particular embodiments, the weight ratio of the sum of the first and second polyglycerol ester to the third polyglycerol ester in the composition ranges from 3:1 to 1:3, particularly from 2:1 to 1:2, more particularly from 1.5:1 to 1:1.5.

In particular embodiments, the weight ratio of the first polyglycerol ester to the second polyglycerol ester in the composition ranges from 1:3 to 3:1, particularly from 1:2 to 2:1, more particularly from 1:2 to 1:1.

In certain embodiments, such as when the composition is an oil-based cosmetic product, the composition comprises between 65.0 % (w/w) and 90.0 % (w/w) of an oil, particularly between 75.0 % (w/w) and 90.0 % (w/w) of an oil; between 2.0 % (w/w) and 10.0 % (w/w) of the first polyglycerol ester; between 1.0 % (w/w) and 5.0 % (w/w) of the second polyglycerol ester; and between 4.0 % (w/w) and 20.0 % (w/w) of the third polyglycerol ester, with % (w/w) based on the total weight of the composition.

In certain embodiments, such as when the composition is an emulsifying composition for use as an ingredient in an oil-based cosmetic product, the composition comprises between 25.0 % (w/w) and 60.0 % (w/w) of an oil, particularly between 35.0 % (w/w) and 50.0 % (w/w) of an oil; between 10.0 % (w/w) and 30.0 % (w/w) of the first polyglycerol ester, particularly between 15.0 % (w/w) and 25 % (w/w) of the first polyglycerol ester; between 5 % (w/w) and 25 % (w/w) of the second polyglycerol ester, particularly between 10 % (w/w) and 20 % (w/w) of the second polyglycerol ester; and between 20 % (w/w) and 45 % (w/w) of the third polyglycerol ester, particularly between 25.0 % (w/) and 35.0 % (w/w) of the third polyglycerol ester, with % (w/w) based on the total weight of the composition.

A second, related aspect of the present application relates to a method for the preparation of a cosmetic oil based product, comprising the step of mixing a first polyglycerol ester, a second polyglycerol ester and a third polyglycerol ester with an oil, wherein the first polyglycerol ester is polyglyceryl-3 dioleate, wherein the second polyglycerol ester is polyglyceryl-3 polyricoleate and wherein the third polyglycerol ester is polyglyceryl-2 laurate, and wherein the sum of the oil and the first, second and third polyglycerol ester is at least 90 % (w/w), or at least 95 % (w/w), based on the total weight of the cosmetic oil-based product. In particular embodiments, the weight ratio of the sum of the first and second polyglycerol ester to the third polyglycerol ester ranges from 3:1 to 1:3, particularly from 2:1 to 1:2, more particularly from 1.5:1 to 1:1.5. In particular embodiments, the weight ratio of the first polyglycerol ester to the second polyglycerol ester ranges from 1:3 to 3:1, particularly from 1:2 to 2:1, more particularly from 1:2 to 1:1.

In particular embodiments, the cosmetic oil-based product comprises between 25.0 % (w/w) and 90.0 % (w/w) of the oil; between 2.0 % (w/w) and 30.0 % (w/w) of the first polyglycerol ester; between 1.0 % (w/w) and 25.0 % (w/w) of the second polyglycerol ester; and between 4.0 wt% and 45.0 % (w/w) of the third polyglycerol ester.

In particular embodiments, the method steps are performed at a temperature ranging between 35 °C and 60 °C, particularly between 40 °C and 50 °C.

A third, related aspect relates to the use of a composition according to the present application as a cleansing oil-based product, such as a shower oil, a bath oil, or a make-up remover, particularly wherein the cleansing oil based product is a spontaneously emulsifying cleansing oil based product when mixed with water. The present application also provides for the use of a composition according to the present application as an ingredient of a cosmetic oil-based product.

### Detailed description

Before the present system and method of the invention are described, it is to be understood that this invention is not limited to particular systems and methods or combinations described, since such systems and methods and combinations may, of course, vary. It is also to be understood that the terminology used he-rein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

In the present application, the percentages are given by weight, unless otherwise stated.

The inventors have surprisingly found that the combination of three specific polyglycerol esters, in particular polyglyceryl-3 dioleate, polyglyceryl-3 polyricoleate and polyglyceryl-2 laurate, results in a composition with emulsifying properties that is a natural alternative for ethoxylated emulsifiers in oil-based cosmetic products, particularly oil-based skincare products. When this combination of polyglycerol esters is present in a cosmetic oil-based product, a clear homogeneous blend is formed which spontaneously emulsifies upon contact with water, and which does not leave a greasy effect after rinsing off the oil-based product.

These effects could not be predicted based on the properties of the individual polyglycerol esters. Indeed, a mixture of polyglyceryl-3 polyricoleate and an oil forms an oily composition that does not emulsify with water. Polyglyceryl-3 dioleate and polyglyceryl-2 laurate do not mix well with oils, and thus do not form a homogeneous mixture. The present application thus generally relates to an emulsifying composition comprising polyglyceryl-3 dioleate, polyglyceryl-3 polyricoleate and polyglyceryl-2 laurate, and the use thereof, to oil-based compositions comprising said combination of polyglyceryl-3 dioleate, polyglyceryl-3 polyricoleate and polyglyceryl-2 laurate, and to methods to prepare said oil-based compositions.

As used herein, an emulsifier or an emulsifying composition is defined as a substance or composition that stabilizes an emulsion. As used herein, emulsions are systems of two or more immiscible liquids or immiscible liquid phases. One of the liquid phases forms the dispersion medium or continuous phase, in which the other phase is distributed in the form of fine droplets. Most emulsions consist of water and oil or fat as immiscible phases. An o/w emulsion or oil-in-water emulsion is a fat-water mixture whose continuous phase is aqueous, whereas a w/o or water-in-oil emulsion is an oil/fat-water mixture whose continuous phase is an oil or another organic liquid that is not miscible with water.

A first aspect of the present application relates to a composition, comprising a first, a second and a third polyglycerol ester, wherein the first polyglycerol ester is polyglyceryl-3 dioleate (CAS number 79665-94-4), wherein the second polyglycerol ester is polyglyceryl-3 polyricoleate (CAS number 29894-35-7) and wherein the third polyglycerol ester is polyglyceryl-2 laurate (CAS number 96499-68-2), and wherein the sum of the first, second and third polyglycerol ester is at least 95 % (w/w), particularly at least 96 % (w/w), at least 97 % (w/w), at least 98 % (w/w), at least 99 % (w/w) or even at least 99.9% (w/w), with % (w/w) based on the total weight of polyglycerol esters in the composition.

As used herein, the term "polyglycerol" refers to a polymer or oligomer consisting of glycerol monomers. In particular, a polyglyceryl-3 ester comprises on average 3 glycerol monomers and a polyglyceryl-2 ester comprises on average 2 glycerol monomers.

In particular embodiments of the compositions envisaged herein, the weight ratio of the sum of the first and second polyglycerol ester to the third polyglycerol ester, or the weight ratio of the sum of polyglyceryl-3 dioleate and polyglyceryl-3 polyricoleate to polyglyceryl-2 laurate ranges from 3:1 to 1:3, particularly from 2:1 to 1:2, more particularly from 1.5:1 to 1:1.5.

In particular embodiments, the weight ratio of the first polyglycerol ester to the second polyglycerol ester, or the weight ratio of polyglyceryl-3 dioleate to polyglyceryl-3 polyricoleate ranges from 1:3 to 3:1, particularly from 1:2 to 2:1, more particularly from 1:2 to 1:1.

In particular embodiments, the composition comprises between 20 and 40 % (w/w) of polyglyceryl-3 dioleate, between 10 and 30 % (w/w) polyglyceryl-3 polyricoleate and 30 and 70 % (w/w) of polygyceryl-2 laurate, even more in particular comprises between 25 and 35% w/w) of polyglyceryl-3 dioleate, between 15 and 30 % (w/w) or between 15 and 25 % (w/w) of polyglyceryl-3 polyricoleate and between 40 and 60 % (w/w) or between 45 and 55 % (w/w) of polyglyceryl-2 laurate, with % (w/w) based on the total weight of polyglycerol esters in the composition.

In particular embodiments, the composition according to the present application further comprises an oil, wherein the sum of the oil and the first, second and third polyglycerol ester is at least 90 % (w/w), or at least 95 % (w/w) or at least 98% (w/w), based on the total weight of the composition.

It is an advantage of the present application that the effects of the use of the specific combination of the first, second and third polyglycerol ester as envisaged herein, in particular the blooming effect, are applicable to a wide variety of oils or combination of oils. Accordingly, the oil is not particularly limited in the composition according to the present application. As used herein, the term "oil" generally refers to a hydrophobic compound or composition that is liquid at room temperature, in particular at temperatures between 10°C and 35°C, and that is not soluble in water. It will be understood that the term "oil" refers to a composition that does not comprise a polyglycerol ester.

The oil may comprise one or more triglycerides of saturated and/or unsaturated fatty acids, such as capric/caprylic triglycerides, a vegetable oil or a mixture of vegetable oils. The oil may also comprise a synthetic ester of a fatty acid, such as isoamyl laurate, ethylhexyl stearate, decyl oleate, coc-caprylate/caprate, isodecyl neopentanoate, c12-15 alkyl benzoate, cetearyl ethylhexanoate, ceteary isononoate, isopropyl mirystate, isopropyl palmitate, isononyl isononaoate, ethylheaxyl cocoate, ethylhexyl palmitate, pentaerythrityl tetracaprylate/tetracaprate, triethyl citrate.

The oil may be a mineral oil, such as paraffin oil or vaseline oil; an oil of vegetable origin, such as sweet-almond oil, jojoba oil, coconut oil, olive oil, argan oil, shea butter, grapeseed oil, avocado oil, sunflower oil, macadamia oil, rapeseed oil, apricot oil, soybean oil, or castor oil; a modified vegetable oil, an oil of animal origin, or a synthetic oil. The oil may also be or comprise an essential oil or a perfume compound. As used herein, the term "essential oil" refers to a hydrophobic liquid, usually concentrated and of natural origin, containing volatile chemical compounds from plants. Natural oils, i.e. non-synthetic oils are particularly preferred.

The term "natural" as used herein indicates an isolated compound, composition or ingredient originating from a natural origin and which remains structurally essentially unchanged from its natural state, or which has undergone some limited processing to improve its stability or efficacy. The term "synthetic" refers to a compound which is not of natural origin.

In particular embodiments, the composition according to the present application comprises between 25.0 % (w/w) and 90.0 % (w/w) of the oil; between 2.0 % (w/w) and 30.0 % (w/w) of the first polyglycerol ester; between 1.0 % (w/w) and 25.0 % (w/w) of the second polyglycerol ester; and between 4.0 % (w/w) and 45.0 % (w/w) of the third polyglycerol ester; wherein the first polyglycerol ester is polyglyceryl-3 dioleate, wherein the second polyglycerol ester is polyglyceryl-3 polyricoleate and wherein the third polyglycerol ester is polyglyceryl-2 laurate; wherein the sum of the first, second and third polyglycerol ester is at least 95 % (w/w) based on the total weight of polyglycerol esters in the composition; and wherein the sum of the oil and the first, second and third polyglycerol ester is at least 90 % (w/w), or at least 95 % (w/w), based on the total weight of the composition.

More in particular, the weight ratio of the sum of the first and second polyglycerol ester to the third polyglycerol ester ranges from 3:1 to 1:3, particularly from 2:1 to 1:2, more particularly from 1.5:1 to 1:1.5. More in particular, the weight ratio of the first polyglycerol ester to the second polyglycerol ester ranges from 1:3 to 3:1, particularly from 1:2 to 2:1, more particularly from 1:2 to 1:1.

In particular embodiments, the composition comprises between 65.0 % (w/w) and 90.0 % (w/w) of an oil, particularly between 75.0 % (w/w) and 90.0 % (w/w) of an oil, as described elsewhere herein; between 2.0 % (w/w) and 10.0 % (w/w) of the first polyglycerol ester; between 1.0 % (w/w) and 5.0 % (w/w) of the second polyglycerol ester; and between 4.0 % (w/w) and 20.0 % (w/w) of the third polyglycerol ester, with % (w/w) based on the total weight of the composition; wherein the first polyglycerol ester is polyglyceryl-3 dioleate, wherein the second polyglycerol ester is polyglyceryl-3 polyricoleate and wherein the third polyglycerol ester is polyglyceryl-2 laurate; wherein the sum of the first, second and third polyglycerol ester is at least 95 % (w/w) based on the total weight of polyglycerol esters in the composition; and wherein the sum of the oil and the first, second and third polyglycerol ester is at least 90 % (w/w), or at least 95 % (w/w), based on the total weight of the composition. More in particular, the weight ratio of the sum of the first and second polyglycerol ester to the third polyglycerol ester ranges from 3:1 to 1:3, particularly from 2:1 to 1:2, more particularly from 1.5:1 to 1:1.5. More in particular, the weight ratio of the first polyglycerol ester to the second polyglycerol ester ranges from 1:3 to 3:1, particularly from 1:2 to 2:1, more particularly from 1:2 to 1:1.

These embodiments of a composition according to the present application are particularly applicable as a cosmetic oil-based product, particularly an oil-based cleansing product, such as a shower oil, a make-up remover, or a bath oil. Advantageously, such compositions are in the form of a clear, homogeneous mixture or blend, will spontaneously emulsify upon contact with water, and do not leave a greasy effect after rinsing.

In other particular embodiments, the composition comprises between 25.0 % (w/w) and 60.0 % (w/w) of an oil, particularly between 35.0 % (w/w) and 50.0 % (w/w) of an oil; between 10.0 % (w/w) and 30.0 % (w/w) of the first polyglycerol ester, particularly between 15.0 % (w/w) and 25 % (w/w) of the first polyglycerol ester; between 5 % (w/w) and 25 % (w/w) of the second polyglycerol ester, particularly between 10 % (w/w) and 20 % (w/w) of the second polyglycerol ester; and between 20 % (w/w) and 45 % (w/w) of the third polyglycerol ester, particularly between 25.0 % (w/) and 35.0 % (w/w) of the third polyglycerol ester, with % (w/w) based on the total weight of the composition; wherein the first polyglycerol ester is polyglyceryl-3 dioleate, wherein the second polyglycerol ester is polyglyceryl-3 polyricoleate and wherein the third polyglycerol ester is polyglyceryl-2 laurate; wherein the sum of the first, second and third polyglycerol ester is at least 95 % (w/w) based on the total weight of polyglycerol esters in the composition; and wherein the sum of the oil and the first, second and third polyglycerol ester is at least 90 % (w/w), or at least 95 % (w/w), based on the total weight of the composition. More in particular, the weight ratio of the sum of the first and second polyglycerol ester to the third polyglycerol ester ranges from 3:1 to 1:3, particularly from 2:1 to 1:2, more particularly from 1.5:1 to 1:1.5. More in particular, the weight ratio of the first polyglycerol ester to the second polyglycerol ester ranges from 1:3 to 3:1, particularly from 1:2 to 2:1, more particularly from 1:2 to 1:1.

These embodiments of a composition according to the present application are particularly applicable as an emulsifying ingredient in the preparation of a cosmetic oil-based product, particularly an oil-based cleansing or skincare product, such as a shower oil, a make-up remover, or a bath oil. Advantageously, the addition of oil to the different polyglycerol esters was found to improve the production process, as it was easier to mix the polyglycerol esters and requires less heat in the process.

The different embodiments of the compositions according to the present application may further comprise additional cosmetically acceptable ingredients or adjuvants, particularly oil-soluble cosmetically acceptable ingredients or adjuvants, such as oil-soluble emollients, perfumes, pigments or dyes, antioxidants, vitamins - such as vitamins A and E, essential oils, anti-aging molecules, preservatives, saturated or unsaturated alcohols, particularly higher alcohols having between 4 and 10 carbon atoms, and the like.

In particular embodiments, the composition according to the present application is free of ethoxylated compounds, particularly free of ethoxylated emulsifiers. In further particular embodiments, the composition according to the present application is free of water. "Free" as used herein means that while it is preferred that the specific component is absent in the composition, i.e. 0 % (w/w), it is possible to have very small or trace amounts of it in the compositions of the invention, i.e. wherein the specific component is present as an impurity, in particular is present in an amount less than about 0.1% (w/w), typically less than about 0.05 % (w/w) or 0.01 t% (w/w), based on the total weight of the composition.

A further, related aspect of the present application provides a method for the preparation of a composition according to the present application, in particular a cosmetic oil-based product, such as a shower oil, make-up remover, or bath oil. The method comprises the step of mixing a first polyglycerol ester, a second polyglycerol ester and a third polyglycerol ester with an oil as described elsewhere herein, wherein the first polyglycerol ester is polyglyceryl-3 dioleate, wherein the second polyglycerol ester is polyglyceryl-3 polyricoleate and wherein the third polyglycerol ester is polyglyceryl-2 laurate, and wherein the sum of the oil and the first, second and third polyglycerol ester is at least 90 % (w/w), or at least 95 % (w/w), based on the total weight of the cosmetic oil-based product. More in particular, the first, second and third polyglycerol esters are mixed in a weight ratio wherein the sum of the first and second polyglycerol ester to the third polyglycerol ester ranges from 3:1 to 1:3, particularly from 2:1 to 1:2, more particularly from 1.5:1 to 1:1.5. More in particular, the first, second and third polyglycerol esters are mixed in a weight ratio, wherein the sum of the first and second polyglycerol ester to the third polyglycerol ester ranges from 3:1 to 1:3, particularly from 2:1 to 1:2, more particularly from 1.5:1 to 1:1.5.

In particular embodiments, the method comprises the step of mixing between 25.0 % (w/w) and 90.0 % (w/w) of the oil; between 2.0 % (w/w) and 30.0 % (w/w) of the first polyglycerol ester; between 1.0 % (w/w) and 25.0 % (w/w) of the second polyglycerol ester; and between 4.0 wt% and 45.0 % (w/w) of the third polyglycerol ester, with %(w/w) based on the total weight of the composition.

Advantageously, the method can be performed at lower temperatures compared to when other emulsifiers are used, such as ethoxylated emulsifier. Accordingly, in particular embodiments, the method is performed at a temperature ranging between 15 °C and 60 °C, particularly between 40 °C and 50 °C, or at room temperature, between 15°C and 35 °C.

A further, related aspect of the present application relates to the use of a composition according to the present application as a cleansing oil-based product, such as a bath oil, a shower oil or a make-up remover, particularly wherein the cleansing oil-based product is a spontaneously emulsifying cleansing oil-based product when mixed with or upon contact with water, and which does not exhibit a greasy feel when rinsed off.

A further, related aspect of the present application relates to the use of a composition according to the present application as an ingredient of a cosmetic oil-based product, particularly as an alternative to ethoxylated emulsifiers.

### Examples

### Example 1 - Preparation of a spontaneously emulsifying composition

Sample "Bloom", a composition according to the present application comprising 40 % (w/w) caprylic/capric triglyceride; 18 % (w/w) polyglyceryl-3 dioleate; 12 % (w/w) polyglyceryl-3 polyricoleate and 30 % (w/w) polyglyceryl-2 laurate was prepared by mixing the individual components. "Bloom" is particularly suitable for addition to many different types of oils as an emulsifying composition, such as in shower oil applications.

For instance, the formulation works with the following oils:

| | Macadamia oil | Jojoba oil | Rapeseed oil | Castor oil | Sunflower oil | Caprylic/Capric Triglyceride |
|---|---|---|---|---|---|---|
| Oil (%) | 75 | 75 | 75 | 75 | 75 | 65 |
| Bloom (%) | 25 | 25 | 25 | 25 | 25 | 35 |

The "Bloom" formulation is an efficient emulsifying composition for oil-based products which in contact with water, such as when applied to wet skin, spontaneously emulsify and turn into a milky texture, an effect referred to as "blooming". This effect could not be predicted based on the properties of the individual components of the "Bloom" formulation. Indeed, a mixture of polyglyceryl-3 polyricoleate and caprylic/capric triglyceride generates a composition that does not emulsify with water. Polyglyceryl-3 Dioleate and Polyglyceryl-2 Laurate do not mix well with caprylic/capric triglyceride and do not form a homogeneous mixture.

The "Bloom" formulation exhibited several advantageous effects, in particular in combination with one or more oils, as can easily be verified empirically by a skilled person, e.g. visually or by touch. More in particular, a model shower oil comprising a mixture of one or more oils and the "Bloom" formulation formed a clear homogeneous blend, which exhibited a high blooming effect, i.e. it emulsified into a milky texture when applied to wet skin upon contact with water, and no greasy residue is left behind after contact with water; indeed there was no greasy feel after rinsing off the product. Furthermore, the "Bloom" formulation was found to work with any oil combination.

### Example 2 - Comparative examples

Other combinations of polyglycerol esters did not result in the effects obtained for the "Bloom" formulation.
A/ A mixture of sunflower oil (60-70%) and a formulation (30-40%) consisting of a 1:1 mixture of polyglycerol-3 polyricinoleate and a second polyglyceryl ester selected from the group of polyglycerol-10 oleate, polyglycerol-4 laurate, polyglyceryl-6 laurate, and polyglyceryl-10 laurate, did not result in a clear, homogeneous blend and did not exhibit the blooming property.
B/ A mixture of caprylic/capric triglyceride (60-70%) and a formulation (30-40%) consisting of a 1:1 mixture of polyglyceryl-2 laurate and a second polyglyceryl ester selected from the group of polyglycerol-10 oleate, polyglycerol-4 laurate, polyglyceryl-4 caprate, and polyglyceryl-10 laurate did not result in a clear, homogeneous blend and did not exhibit the blooming property.
C/ A composition comprising grape seed oil (24%), almond oil (20%), olive oil (12%), macadamia oil (10-14.5 %), moringa oil (0.5- 5 %), rose hip oil (13.8 %) and polyglyceryl-4 oleate (15%) formed a clear, homogeneous blend. However, it required heating during preparation of the composition, and the composition exhibited little or even no blooming effect and left a greasy feel.

## Claims

1. A composition, particularly a homogeneous composition, comprising a first, a second and a third polyglycerol ester, wherein the first polyglycerol ester is polyglyceryl-3 dioleate, wherein the second polyglycerol ester is polyglyceryl-3 polyricoleate and wherein the third polyglycerol ester is polyglyceryl-2 laurate, and wherein the sum of the first, second and third polyglycerol ester is at least 95 % (w/w) based on the total weight of polyglycerol esters in the composition.

2. The composition according to claim 1, further comprising an oil, wherein the sum of the oil and the first, second and third polyglycerol ester is at least 90 % (w/w), or at least 95 % (w/w), based on the total weight of the composition.

3. The composition according to claim 2, wherein the composition comprises
- between 25.0 % (w/w) and 90.0 % (w/w) of the oil;
- between 2.0 % (w/w) and 30.0 % (w/w) of the first polyglycerol ester;
- between 1.0 % (w/w) and 25.0 % (w/w) of the second polyglycerol ester; and
- between 4.0 % (w/w) and 45.0 % (w/w) of the third polyglycerol ester.

4. The composition according to any one of claims 1 to 3, wherein the weight ratio of the sum of the first and second polyglycerol ester to the third polyglycerol ester ranges from 3:1 to 1:3, particularly from 2:1 to 1:2, more particularly from 1.5:1 to 1:1.5.

5. The composition according to any one of claims 1 to 4, wherein the weight ratio of the first polyglycerol ester to the second polyglycerol ester ranges from 1:3 to 3:1, particularly from 1:2 to 2:1, more particularly from 1:2 to 1:1.

6. The composition according to any one of claims 1 to 5, wherein the composition comprises:
- between 65.0 % (w/w) and 90.0 % (w/w) of an oil, particularly between 75.0 % (w/w) and 90.0 % (w/w) of an oil;
- between 2.0 % (w/w) and 10.0 % (w/w) of the first polyglycerol ester;
- between 1.0 % (w/w) and 5.0 % (w/w) of the second polyglycerol ester; and
- between 4.0 % (w/w) and 20.0 % (w/w) of the third polyglycerol ester.
with % (w/w) based on the total weight of the composition.

7. The composition according to any one of claims 1 to 5, wherein the composition comprises:
- between 25.0 % (w/w) and 60.0 % (w/w) of an oil, particularly between 35.0 % (w/w) and 50.0 % (w/w) of an oil;
- between 10.0 % (w/w) and 30.0 % (w/w) of the first polyglycerol ester, particularly between 15.0 % (w/w) and 25 % (w/w) of the first polyglycerol ester;
- between 5 % (w/w) and 25 % (w/w) of the second polyglycerol ester, particularly between 10 % (w/w) and 20 % (w/w) of the second polyglycerol ester; and
- between 20 % (w/w) and 45 % (w/w) of the third polyglycerol ester, particularly between 25.0 % (w/) and 35.0 % (w/w) of the third polyglycerol ester,
with % (w/w) based on the total weight of the composition.

8. A method for the preparation of a cosmetic oil product, comprising the step of mixing a first polyglycerol ester, a second polyglycerol ester and a third polyglycerol ester with an oil, wherein the first polyglycerol ester is polyglyceryl-3 dioleate, wherein the second polyglycerol ester is polyglyceryl-3 polyricoleate and wherein the third polyglycerol ester is polyglyceryl-2 laurate, and wherein the sum of the oil and the first, second and third polyglycerol ester is at least 90 % (w/w), or at least 95 % (w/w), based on the total weight of the cosmetic oil product.

9. The method according to claim 8, wherein the weight ratio of the sum of the first and second polyglycerol ester to the third polyglycerol ester ranges from 3:1 to 1:3, particularly from 2:1 to 1:2, more particularly from 1.5:1 to 1:1.5.

10. The method according to claim 8 or 9, wherein the weight ratio of the first polyglycerol ester to the second polyglycerol ester ranges from 1:3 to 3:1, particularly from 1:2 to 2:1, more particularly from 1:2 to 1:1.

11. The method according to any one of claims 8 to 10, wherein the cosmetic oil product comprises between 25.0 % (w/w) and 90.0 % (w/w) of the oil; between 2.0 % (w/w) and 30.0 % (w/w) of the first polyglycerol ester; between 1.0 % (w/w) and 25.0 % (w/w) of the second polyglycerol ester; and between 4.0 wt% and 45.0 % (w/w) of the third polyglycerol ester.

12. The method according to any one of claims 8 to 11, wherein the method is performed at a temperature ranging between 35 °C and 60 °C, particularly between 40 °C and 50 °C.

13. Use of a composition according to any one of claims 1 to 7 as a cleansing oil product, such as a bath oil, a shower oil or a make-up remover, particularly wherein the cleansing oil product is a spontaneously emulsifying cleansing oil product when mixed with water.

14. Use of a composition according to any one of claims 1 to 7 as an ingredient of a cosmetic oil product.
